# EUROPÄISCHE PATENTSCHRIFT

(11) **EP 1 307 733 B1**
(45) Veröffentlichungstag und Bekanntmachung des Hinweises auf die Patenterteilung: **12.10.2005**
(21) Anmeldenummer: 01957622.2
(22) Anmeldetag: 09.08.2001
(51) Int. Cl.: G01N 27/327, C12Q 1/00

(54) **CREATININSENSOR-KALIBRATION**
CREATININE SENSOR CALIBRATION
ETALONNAGE DE DETECTEUR DE CREATININE

(30) Priorität: 11.08.2000 AT 13912000
(43) Veröffentlichungstag der Anmeldung: 07.05.2003
(73) Patentinhaber: F. HOFFMANN-LA ROCHE AG, 4070 Basel (CH); Roche Diagnostics GmbH, 68305 Mannheim (DE)
(72) Erfinder: SCHAFFAR, Bernhard, A-8045 Graz (AT); KRONEIS, Herbert, A-8052 Graz (AT); NOORMOFIDI, Taghi, A-8054 Graz (AT); FLORIAN, Gernot, A-8010 Graz (AT); STEINBÖCK, Wolf-Dietrich, A-8020 Graz (AT)
(74) Vertreter: Schwarz, Albin
(86) Internationale Anmeldenummer: PCT/AT2001/000265
(87) Internationale Veröffentlichungsnummer: WO 2002/014533

(56) Entgegenhaltungen:
- GB-A- 2 185 318
- PATENT ABSTRACTS OF JAPAN vol. 007, no. 150 (P-207), 30. Juni 1983 (1983-06-30) & JP 58 061459 A (HITACHI SEISAKUSHO KK), 12. April 1983 (1983-04-12)
- PATENT ABSTRACTS OF JAPAN vol. 009, no. 327 (P-415), 21. Dezember 1985 (1985-12-21) & JP 60 151560 A (FUJI DENKI SOUGOU KENKYUSHO:KK), 9. August 1985 (1985-08-09)

## Beschreibung

Die Erfindung betrifft ein Verfahren zur Kalibration eines Biosensors zur amperometrischen Bestimmung von Creatinin in biologischen Flüssigkeiten, bestehend aus jeweils einem Elektrodensystem zur Messung der Creatinin- und Creatinkonzentration, wobei mindestens zwei Kalibrationslösungen benutzt werden.

Die Bestimmung enzymatisch abbaubarer Stoffe, wie Creatinin, Glucose, etc., mittels Sensoren in biologischen Flüssigkeiten, zum Beispiel in Blut, Urin, Plasma, Serum und Liquor, erfolgt vorzugsweise über Biosensoren mit immobilisierten Enzymen. In der Literatur sind mehrere elektrochemische und photometrische Verfahren zur Bestimmung dieser Stoffe bekannt.

So kann beispielsweise über das Enzym Creatinine Deiminase mit nachfolgender Bestimmung des Ammoniumgehalts Creatinin potentiometrisch bestimmt werden. Ein anderes Verfahren besteht darin, die Creatininkonzentration über eine Enzymkaskade unter Verwendung der Enzyme Creatininase, Creatinase und Sarkosinoxidase zu bestimmen, wobei letztendlich Wasserstoffperoxid (H₂O₂) an einer amperometrischen Elektrode gemessen wird (Tsuchida, T., Yoda, K., Clin.Chem. 29/1, 51-55 (1983)).

Die Erfindung bezieht sich auf ein Verfahren zur Kalibration von Biosensoren, die nach dem letztgenannten Prinzip arbeiten. Die Umsetzung von Creatinin zu Wasserstoffperoxid erfolgt nach den folgenden Reaktionsschritten:

| | | |
|---|---|---|
| Creatinin + H₂O | - Creatininase → | Creatin |
| Creatin + H₂O | - Creatinase → | Sarkosin + Harnstoff |
| Sarkosin + O₂ + H₂O | - Sarkosinoxidase → | Glycin + Formaldehyd + H₂O₂ |

Um Creatinin in einer biologischen Probe einwandfrei bestimmen zu können, ist es notwendig, die Ergebnisse des Creatininsystems, das aus einem Elektrodensystem mit allen drei Enzymen besteht, um jene des Creatinsystems, dessen Elektrodensystem nur die Enzyme Creatinase und Sarkosinoxidase umfaßt, zu korrigieren, da in biologischen Proben meistens Creatinin und Creatin nebeneinander vorkommen und das Creatininsystem systembedingt nur die Summe aus Creatinin und Creatin erkennen kann.

Für eine Kalibration des Biosensors sind somit sowohl Lösungen zur Kalibrierung des Creatinin- als auch des Creatinsystems nötig.

Erschwert wird die Kalibration eines Biosensors zur Bestimmung von Creatinin weiters dadurch, daß Creatinin und Creatin bei Raumtemperatur und neutralem pH-Wert der Lösung im Gleichgewicht stehen, d.h. keiner der beiden Analyten in Lösung für sich allein in stabiler Konzentration gehalten werden kann, was für eine Kalibration der Elektrodensysteme jedoch unbedingt notwendig wäre.

Der Benützer des Biosensors ist daher gezwungen, die Kalibrationslösung(en) jeweils durch Einwägen von Creatinin und/oder Creatin in einen Behälter und Lösen der Einwaage in einer bestimmten Menge an Pufferflüssigkeit vor einer Messung frisch zuzubereiten. Zwar ist (sind) diese Lösung(en) dann bei Raumtemperatur für eine kurze Zeitspanne, im allgemeinen etwa eine Woche, ausreichend stabil, da die Umwandlung von Creatinin in Creatin bzw. umgekehrt sehr langsam vor sich geht, doch ist der Vorgang der Zubereitung nach dieser Zeitspanne zu wiederholen.

Diese Methode ist nachteilig, da die ständige, zumindest wöchentliche, frische Zubereitung der Kalibrationslösungen eine jederzeit rasche, einfache und örtlich flexible Durchführung der Kalibrierung verhindert. Überdies bedingt das wiederholte Einwägen und Lösen zwecks Sicherstellung einer ausreichenden Genauigkeit einen höheren Verbrauch an Creatinin bzw. Creatin und Pufferflüssigkeit.

Die Erfindung stellt sich die Aufgabe, ein Verfahren der eingangs genannten Art zu schaffen, das die oben genannten Nachteile überwindet. Insbesondere soll das erfindungsgemäße Verfahren Kalibrationslösungen bereitstellen, die nicht wöchentlich durch Einwägen hergestellt werden müssen und zu deren Herstellung bezogen auf die Zahl der Anwendungen im Vergleich zum Stand der Technik weniger Ausgangsstoff notwendig ist. Die Herstellung der Kalibrationslösungen soll zudem rasch, einfach und praktisch überall durchgeführt werden können.

Diese Aufgabe wird erfindungsgemäß dadurch gelöst, daß das Elektrodensystem zur Messung der Creatininkonzentration mit einer Creatinin-Lösung kalibriert wird, welche vor der Kalibrationsmessung in Form einer sauren Lösung mit einer alkalischen Pufferlösung gemischt wurde, und das Elektrodensystem zur Messung der Creatinkonzentration mit einer Lösung kalibriert wird, in der sich Creatinin und Creatin im thermodynamischen Gleichgewicht befinden.

Aus J.Am.Chem.Soc. 47, 1179-1188, 1925 (G. Edgar, H. E. Shiver) ist bekannt, daß sich das Gleichgewicht Creatin/Creatinin mit zunehmender Temperatur und Wasserstoffionen-Konzentration in Richtung Creatinin verschiebt.

Gemäß Biochem.J., 920-929,1928 (R. K. Cannan, A. Shore) ist die Geschwindigkeit der Gleichgewichtsreaktion Creatin ↔ Creatinin in stark saurer Lösung proportional der Wasserstoffionen-Konzentration. In sauren Lösungen um pH 3 (z.B. 2<pH<4) ist die Umwandlung von Creatin in Creatinin im wesentlichen irreversibel.

Die Erfindung macht sich die Tatsache zunutze, daß Creatinin in Lösung im wesentlichen stabil gehalten werden kann, wenn der pH-Wert der Lösung im sauren Bereich liegt. Mit einer solchen Lösung können zwar Biosensoren nicht mehr kalibriert werden, da sowohl die Aktivität als auch die Lebensdauer des Sensors in derart saurem Milieu stark beeinträchtigt ist, doch kann durch Mischen einer kleinen Menge dieser sauren Lösung mit einer aus leicht alkalischen Puffersalzen bestehenden Lösung einfach eine Creatininkalibrationslösung hergestellt werden, die bei Raumtemperatur ebenfalls zumindest für eine Woche ausreichend stabil ist und danach durch eine neue Mischung von saurer Creatinin-Lösung und alkalischer Pufferlösung ersetzt werden kann, ohne eine Einwaage von Creatinin vornehmen zu müssen.

Vorzugsweise wird als saure Lösung eine Creatinin-Lösung mit einem pH-Wert von 2-4, eher bevorzugt von 2,5-3, eingesetzt.

Die Mischung der sauren Creatinin-Lösung und der alkalischen Pufferlösung kann beispielsweise automatisch in dem Gerät erfolgen, in welchem der Biosensor integriert ist und betrieben wird. Zu diesem Zweck sind lediglich in größeren Abständen die für die Aufnahme der Lösungen vorgesehenen Behälter mit frisch zubereiteten Lösungen aufzufüllen.

Mit Hilfe der erfindungsgemäß hergestellten Creatininkalibrationslösung kann das Elektrodensystem zur Messung der Creatininkonzentration eindeutig kalibriert werden. Eventuell sich nach einiger Zeit bildendes Creatin kann durch Bestimmung mittels eines bereits kalibrierten Sensors gesondert bestimmt und in Abzug gebracht werden.

Das Elektrodensystem zur Messung der Creatinkonzentration hingegen wird mit Hilfe einer neutralen Lösung kalibriert, in welcher Creatinin und Creatin mit den für eine bestimmte Temperatur bekannten Gleichgewichtskonzentrationen vorliegen. Zusätzlich ist mit dieser Lösung der Einfluß des Creatins auf das Creatininsystem indirekt bestimmbar bzw. kalibrierbar.

Vorzugsweise wird die genaue Creatininkonzentration der Creatininkalibrationslösung nach dem Mischen von Creatinin-Lösung und Pufferlösung mit Hilfe eines Leitions, das nur in einer der beiden Mischungskomponenten eingewogen wurde, berechnet. Indem das Mischungsverhältnis mit dem Leition überprüft wird, kann über die bekannte Einwaagekonzentration der exakte Creatiningehalt der Mischung, also der Kalibrationslösung, berechnet werden.

Es ist weiters vorteilhaft, wenn den Kalibrationslösungen Interferenten, die in den zu untersuchenden Flüssigkeiten vorhanden sind, beispielsweise Enzyminhibitoren, und/oder Inhibitoren, welche eine ähnliche Wirkung auf die Enzyme haben wie die in den zu untersuchenden Flüssigkeiten vorhandenen - solche Inhibitoren haben den Vorteil, in Reagenzien stabil eingebracht werden zu können -, zugesetzt werden und zwar in deren durchschnittlicher Konzentration, um deren Einflüsse auf die Meßergebnisse mitberücksichtigen zu können und dadurch das Meßresultat zu verbessern.

Viele biologische Flüssigkeiten enthalten Bicarbonat. Aus J.Mol.Biol. 214, 597-610, 1990 (M. Coll et al) ist bekannt, daß Bicarbonat als Inhibitor der Creatinase wirkt. Es ist daher vorteilhaft, den Kalibrationslösungen Bicarbonat als Interferent, d.h. kompetitiver Inhibitor, zuzusetzen.

Es hat sich jedoch gezeigt, daß das Messverhalten von Biosensoren besonders gut ist, wenn den Kalibrationslösungen Acetat als kompetitiver Inhibitor zugesetzt wird. Im Unterschied zu Bicarbonat kann Acetat vorteilhaft bei neutralem pH-Wert stabil in Lösung gehalten werden.

In den Tabellen 8-10 ist die Auswirkung von Bicarbonat- und Acetatinterferenz in den Kalibrationslösungen auf das Messverhalten von Creatin- und Creatininsensoren veranschaulicht. Tabelle 8 zeigt die Messergebnisse für eine wässrige Lösung, bestehend aus ca. 120 µM Creatinin und 210 µM Creatin, entsprechend einem Creatininverhältnis Cal 2 (siehe Beispiel), welche a) ohne Interferenten, b) mit einem 40 mmol/l - Acetat-Zusatz und c) einem 25 mmol/l - Bicarbonat-Zusatz einer Messung unterzogen wurde. Gemessen wurde einerseits mit einem Biosensor, der mit 40 mmol/l Acetat-Zusatz kalibriert wurde, und andererseits mit einem Sensor, der ohne Acetat-Zusatz kalibriert wurde.

Analog dazu zeigt Tabelle 9 die Messergebnisse der Creatininsensoren für eine wässrige Lösung, enthaltend 230 µM Creatinin, entsprechend einer Kalibrationslösung Cal 1 (siehe weiter unten).

Beide Tabellen zeigen, daß die Bicarbonatinterferenz in der Messlösung sehr gut durch Acetat kompensiert werden kann. Unterstrichen wird diese Erkenntnis auch durch die Ergebnisse von Tabelle 10. Hier wurden verschiedene Bicarbonat-hältige Kontrollmittel mit den oben erwähnten Biosensoren, welche mit und ohne Acetat-Zusatz kalibriert wurden, gemessen. Die Messergebnisse der mit Acetat-Zusatz kalibrierten Sensoren liegen im Durchschnitt um ca. 40 % höher und stimmen besser mit den Sollwerten der Kontrollmittel überein als jene der ohne Acetat-Zusatz kalibrierten Sensoren.

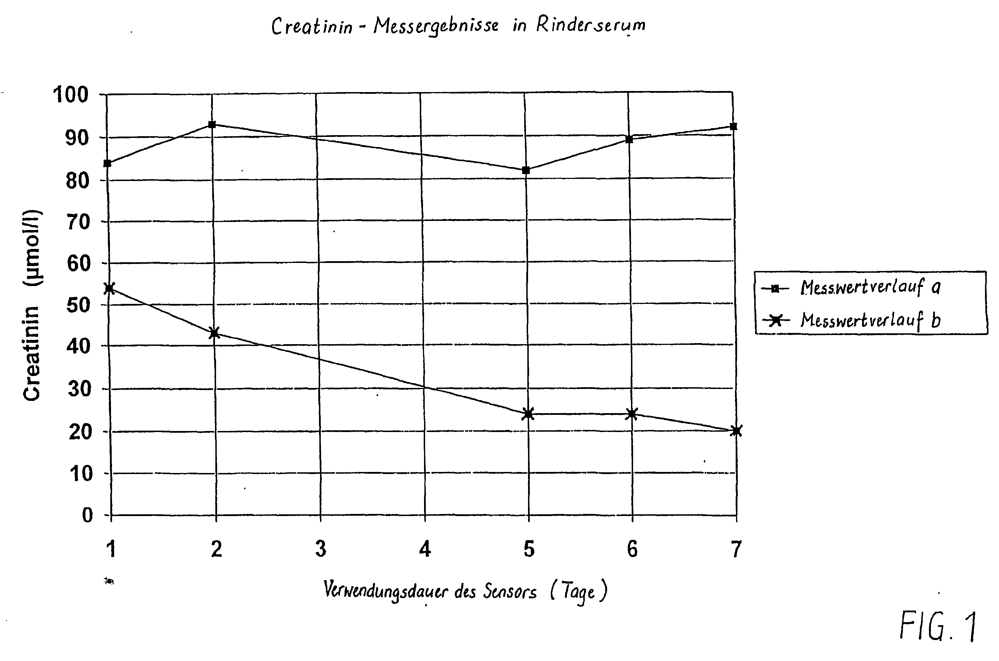

Fig. 1 zeigt a) den Messwertverlauf für einen Creatinin-Sensor, der mit einer 40 mM Acetat enthaltenden Lösung kalibriert wurde, und b) den Messwertverlauf eines ohne Inhibitor-Zusatz zur Kalibrationslösung kalibrierten Creatinin-Sensors über einen Zeitraum von einer Woche. Gemäß Messwertverlauf b wird die Creatinin-Konzentration von etwa 90 µM in der Messlösung (Bicarbonat-hältiges Rinderserum) bereits am ersten Tag mit nur mehr 54 µM bestimmt, was auf eine nicht-kompensierte Interferenz bei der Kalibration des Sensors sowie den natürlichen Abbau des Enzyms zurückzuführen ist, und sinkt im Verlauf einer Woche auf etwa 20 µM. Der Messwertverlauf a hingegen bleibt innerhalb einer Woche in einem Bereich von 82-92 µM, was sehr gut mit dem wahren Wert übereinstimmt.

Zweckmäßig werden die Kalibrationslösungen mit Bioziden, wie Isothiazolon-Derivaten, und/oder mit einer Kombination von Bioziden und Antibiotika versetzt, welche Zusätze eine Stabilisierung der Lösungen bewirken. Hierbei einzusetzende Isothiazolon-Derivate sind beispielsweise 2-Methyl-2,3-dihydroisothiazol-3-on, 5-Chlor-2-methyl-2,3-dihydroisothiazol-3-on oder 2-n-Octyl-2,3-dihydroisothiazol-3-on. Verwendbare Antibiotika sind zum Beispiel Ciprofloxacin oder Gentamicin. Die Sensorsysteme werden bei einer Anwendung dieser Stoffe in wirksamen Konzentrationen nicht beeinträchtigt.

Als Puffer für alkalische Pufferlösungen werden bevorzugt Amin-Puffer nach Norman Good oder Bicarbonat eingesetzt. Als Puffer für saure Lösungen eignen sich Lactat/Milchsäure (siehe z.B. Tabelle 1) und Amin-Puffer nach Norman Good, sofern diese Sulfonsäuregruppen aufweisen (z.B. HEPES oder MOPS; siehe Tabellen 2 und 3)

Durch Variation von Elektrolyt- und Puffersystemen in den Kalibrationslösungen können physiologische Bedingungen, wie pH-Wert, Pufferkapazität, Ionenmilieu und Ionenstärke beliebig an die der zu untersuchenden biologischen Flüssigkeiten angepaßt werden.

Die Erfindung wird nachstehend durch das folgende Beispiel weiter veranschaulicht:

### Beispiel

Formulierung von Kalibrationslösungen zur Kalibration von Creatinin- und Creatinsystemen bzw. der elektrochemischen Interferenz

In den nachfolgenden Tabellen 1-6 werden Varianten für die Formulierung von Kalibrationslösungen angeführt, welche auf die Kalibration von Creatinin und Creatin abgestimmt sind.
Die Kalibrationslösung für die Creatinin-Kalibration ergibt sich aus:
Creatinin-Lösung Cal 1 (sauer) und Pufferlösung Cal 1 (basisch) nach 1:1-Vermischung (Prüfung des Mischungsverhältnisses mit K⁺)
Die Kalibrationslösung für die Creatin-Kalibration ist in den Tabellen mit Cal 2 bezeichnet.

Die in den Tabellen 4 -6 angeführten Formulierungen sind auch zur Kalibration anderer in biologischen Flüssigkeiten vorkommender Stoffe (wie angegeben) verwendbar.

Variante 2 (Cal 2) ist beispielsweise vorteilhaft, wenn Kalibrationskomponenten einzuschließen sind, die entweder im Sauren oder im Basischen stabiler sind als in einer fertigen Kalibrationslösung (z.B. Glucose). Ein weiterer Vorteil dieser Variante besteht darin, daß die Konzentrationsverhältnisse zwischen Creatinin und Creatin variiert werden können.

Auch bei Variante 3 (Cal 1) sind mehr Freiheitsgrade zur Anpassung der Kalibrationslösung an physiologische Verhältnisse möglich.

Beispiele für die zur Kalibrierung eines Sensors außerdem benötigte Wasch- oder Nullpunktskalibrationslösung, die keine der zu bestimmenden Stoffe enthält und somit das "Hintergrundsignal" bzw. die "Nullpunktlinie" des Sensors ermitteln läßt, sind in Tabelle 7 angegeben. Die mengenmäßige Zusammensetzung dieser Lösung kann wie bei den Kalibrationslösungen den in der biologischen Probe vorzufindenden Bedingungen angepaßt werden, wie die angegebenen Varianten zeigen.

Zur Stabilisierung der für die Creatininmessung nötigen Enzyme enthält die Standby-Lösung vorzugsweise MgNa₂EDTA, wobei hierbei eine Menge von 0,5 mM ausreichend ist. Es sind aber auch Mengen bis zu 5 mM MgNa₂EDTA zur Stabilisierung denkbar. Bei Kombinationsanalysegeräten, die auch eine Messung von Ca und Mg durchführen, können sich größere Mengen an MgNa₂EDTA jedoch nachteilig auswirken. Anstelle von MgNa₂EDTA kann auch Hypophosphit als Stabilisator eingesetzt werden (Tsuchida, T, Yoda, K., Clin.Chem. 29/1, 51-55 (1983)).

Die Lösung gemäß Variante 1 kann zusätzlich als Kalibrationslösung für die Kalibration von NH₄⁺- und Urea-Sensoren Anwendung finden. Die anderen Parameter (Ionenstärke, Ionenmilieu, etc.) sind den physiologischen Normalwerten angenähert; die Pufferkapazität ist erhöht.

Variante 2 enthält ebenfalls in ihrer Menge den physiologischen Normalwerten angepaßte Lösungsbestandteile, wobei die Pufferkapazität geringer als bei Variante 1 ist.

Bei Variante 3 sind Pufferkapazität und Ionenstärke deutlich erhöht.

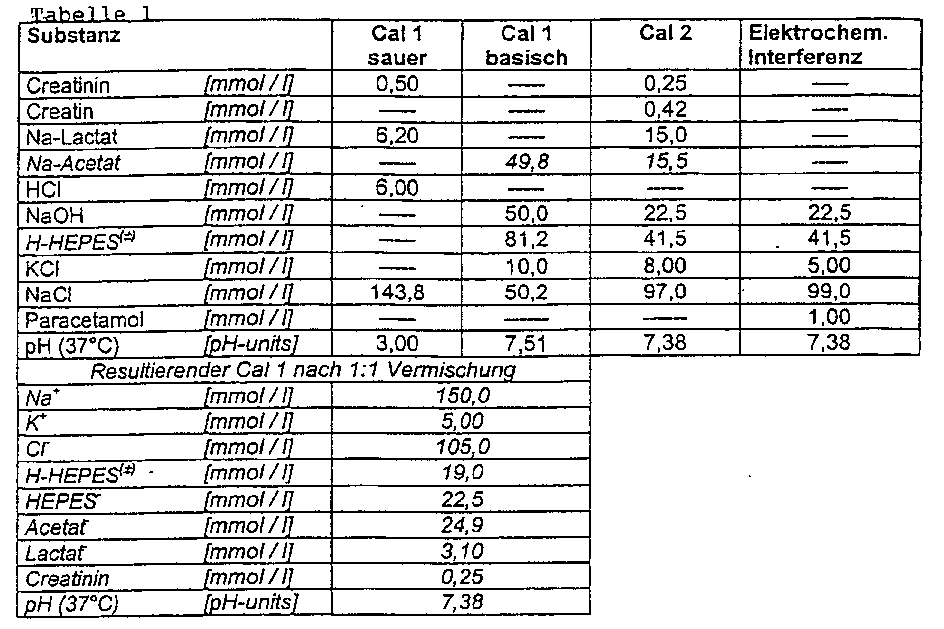

**Tabelle 2**

| *Mögliche Varianten für Cal 1 (sauer)und Cal 1 (basisch): Variante 1 (Cal 1): zur Kalibration von Creatinin* | | | | | | |
|---|---|---|---|---|---|---|
| ***Substanz*** | | ***Cal 1 sauer*** | ***Cal 1 basisch*** | ***Resultierende Lösung nach 1 : 1 - Vermischung*** | | |
| *NaCl* | *[mmol* / *l]* | *150* | *98,4* | *Na*^{*+*} | *[mmol* / *l]* | *150, 0* |
| *KCl* | *[mmol* / *l]* | ----- | *10,0* | *K*^{*+*} | *[mmol* / *l]* | *5,00* |
| *HCl* | *[mmol* / *l]* | *6, 60* | ----- | *Cl*^{*-*} | *[mmol* / *l]* | *132,5* |
| *NaOH* | *[mmol* / *l]* | ----- | *51,6* | *Hepes*^{*-*} | *[mmol* / *l]* | *22,5* |
| *H-HEPES*^{*(±)*} | *[mmol*/*l]* | *20* | *63,0* | *H-HEPES*^{*(±)*} | *[mmol* / *l]* | *19,0* |
| *Creatinin* | *[mmol* / *l]* | *0, 50* | ----- | *Creatinin* | *[mmol* / *l]* | *0, 25* |
| *pH (37°C)* | *[pH-units]* | *2,50* | *7.96* | *pH (37°C)* | *[pH-units]* | *7,38* |

**Tabelle 3**

| *Variante 2 (Cal 1): zur Kalibration von Creatinin* | | | | | | |
|---|---|---|---|---|---|---|
| ***Substanz*** | | ***Cal 1 sauer*** | ***Cal 1 basisch*** | ***Resultierende Lösung nach 1 : 1 - Vermischung*** | | |
| *NaCl* | *[mmol* / *l]* | *150* | *43,4* | *Na*^{*+*} | *[mmol* / *l]* | *150,0* |
| *Na-Acetat* | *[mmol* / *l]* | ----- | *55, 0* | *K*^{*+*} | *[mmol* / *l]* | *5,00* |
| *KCl* | *[mmol* / *l]* | ----- | *10,0* | *Cl*^{*-*} | *[mmol* / *l]* | *105,0* |
| *HCl* | *[mmol* / *l]* | *6, 60* | ----- | *Acetat*^{*-*} | *[mmol* / *l]* | *27,5* |
| *NaOH* | *[mmol* / *l]* | ----- | *51,6* | *Hepes*^{*-*} | *[mmol* / *l]* | *22,5* |
| *H-HEPES*^{*(±)*} | *[mmol* / *l]* | *20* | *63,0* | *H-HEPES*^{*(±)*} | *[mmol* / *l]* | *19,0* |
| *Creatinin* | *[mmol* / *l]* | *0,50* | *-----* | *Creatinin* | *[mmol* / *l]* | *0,25* |
| *pH (37°C)* | *[pH-units]* | *2,50* | *7,96* | *pH (37°C)* | *[pH-units]* | *7,38* |

**Tabelle 4**

| *Variante 3: zur Kalibration von Creatinin, Glucose, Lactat, Harnstoff (pH, Na*^{*+*}*, Cl*^{*-*}*,PCO*_{*2*}*) (Cal 1) K*^{*+*} *dient zur Prüfung, Bestimmung oder Steuerung des Mischungsverhältnisses* | | | | | | |
|---|---|---|---|---|---|---|
| ***Substanz*** | | ***Cal 1 sauer*** | ***Cal 1 basisch*** | ***Resultierende Lösung nach 1 : 1 - Vermischung*** | | |
| *NaCl* | *[mmol* / *l]* | *145,0* | *46,80* | *Na*^{*+*} | *[mmol* / *l]* | *150,0* |
| *Na-Lactat* | *[mmol* / *l]* | *6,0* | ----- | *K*^{*+*} | *[mmol* / *l]* | *5,00* |
| *NaHCO*_{*3*} | *[mmol* / *l]* | ----- | *47, 2* | *NH*_{*4*}^{*+*} | *[mmol* / *l]* | *0,05* |
| *NaOH* | *[mmol* / *l]* | ----- | *55,0* | *Cl*^{*-*} | *[mmol* / *l]* | *107,2* |
| *KCl* | *[mmol* / *l]* | ----- | *10,00* | *Lactat*^{*-*} | *[mmol* / *l]* | *3,00* |
| *HCl* | *[mmol* / *l]* | *12,45* | ----- | *HCO*_{*3*}^{*-*} | *[mmol* / *l]* | *22,34* |
| *NH*_{*4*}*Cl* | *[mmol* / *l]* | *0,10* | ----- | *Hepes*^{*-*} | *[mmol* / *l]* | *22,5* |
| *H-HEPES*^{*(±)*} | *[mmol* / *l]* | *40, 2* | *42,8* | *H-HEPES*^{*(±)*} | *[mmol* / *l]* | *19,0* |
| *Creatinin* | *[mmol* / *l]* | *0,50* | ----- | *Creatinin* | *[mmol* / *l]* | *0,25* |
| *Glucose* | *[mmol* / *l]* | *10,0* | ----- | *Glucose* | *[mmol* / *l]* | *5,00* |
| *Harnstoff* | *[mmol* / *l]* | ----- | *18,0* | *Harnstoff* | *[mmol* / *l]* | *9,00* |
| *pH (37°C)* | *[pH-units]* | *2,90* | *10,13* | *pH (37°C)* | *[pH-units]* | *7,38* |
| | | | | *PCO*_{*2*} *(37°C)* | *[mmHg]* | *40,7* |

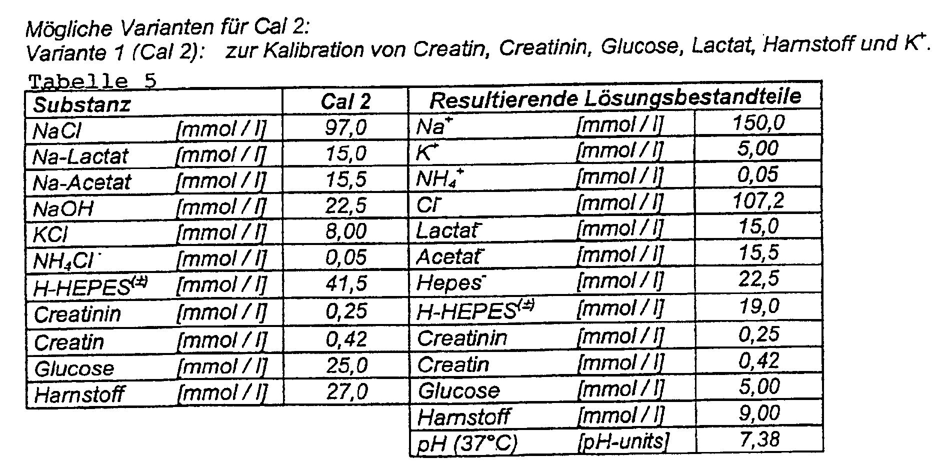

**Tabelle 6**

| *Variante 2 (Cal2): zur Kalibration von Creatin, Creatinin und Glucose.* | | | | | | |
|---|---|---|---|---|---|---|
| ***Substanz*** | | ***Cal*** **2** ***sauer*** | **Cal 2** ***basisch*** | ***Resultierende Lösung nach 1 : 1 - Vermischung*** | | |
| *NaCl* | *[mmol* / *l]* | *150* | *101,7* | *Na*^{*+*} | *[mmol* / *l]* | *150,0* |
| *KCl* | *[mmol* / *l]* | ----- | *10,0* | *K*^{*+*} | *[mmol* / *l*] | *5,00* |
| *HCl* | *[mmol* / *l]* | *3,30* | ----- | *Cl*^{*-*} | *[mmol* / *l]* | *132,5* |
| *NaOH* | *[mmol* / *l]* | ----- | *48,3* | *Hepes*^{*-*} | *[mmol* / *l]* | *22,5* |
| *H-HEPES*^{*(±)*} | *[mmol* / *l]* | *10* | *73, 0* | *H-HEPES*^{*(±)*} | *[mmol* / *l]* | *19,0* |
| *Glucose* | *[mmol* / *l]* | *50, 0* | *-----* | *Glucose* | *[mmol* / *l]* | *25,0* |
| *Creatinin* | *[mmol* / *l]* | *0,21* | *0,29* | *Creatinin* | *[mmol* / *l]* | *0,25* |
| *Creatin* | *[mmol* / *l]* | ----- | *0,50* | *Creatin* | *[mmol* / *l]* | *0,25* |
| *pH (37°C)* | *[pH-units]* | *2,50* | *7,60* | *pH (37°C)* | *[pH-units]* | *7,38* |

**Tabelle 7**

| **Wasch- und Nullpunktskalibrationslösung ("Standby")** | | | | |
|---|---|---|---|---|
| **Substanz** | | **Var. 1** | **Var. 2** | **Var. 3** |
| Creatinin | [mmol / l] | 0,00 | 0,00 | 0,00 |
| Creatin | [mmol / l] | 0,00 | 0,00 | 0,00 |
| H-HEPES^{(±)} | [mmol / l] | 83,0 | 41,5 | 83,0 |
| NaOH | [mmol / l] | 45,0 | 22,5 | 45,0 |
| NaCl | [mmol / l] | 99,95 | 100,0 | 100,0 |
| Na-Acetat | [mmol / l] | 4,05 | 26,5 | 40,0 |
| KCl | [mmol / l] | 5,00 | 5,00 | 5,00 |
| Harnstoff | [mmol / l] | 1,00 | ----- | ----- |
| NH₄Cl | [mmol / l] | 0,05 | ----- | ----- |
| Na₂MgEDTA | [mmol / l] | 0,50 | 0,50 | 0,50 |

| Resultierende Gleichgewichtskonzentrationen | | | | |
|---|---|---|---|---|
| Creatinin | [mmol / l] | 0,00 | 0,00 | 0,00 |
| Creatin | [mmol / l] | 0,00 | 0,00 | 0,00 |
| Na⁺ | [mmol / l] | 150,0 | 150,0 | 186,0 |
| K⁺ | [mmol / l] | 5,00 | 5,00 | 5,00 |
| Mg⁺⁺ | [mmol / l] | 0,50 | 0,50 | 0,50 |
| NH₄⁺ | [mmol / l] | 0,05 | 0,00 | 0,00 |
| Cl⁻ | [mmol / l] | 105,0 | 105,0 | 105,0 |
| H-HEPES^{(±)} | [mmol / l] | 38,0 | 19,0 | 38,0 |
| HEPES⁻ | [mmol / l] | 45,0 | 22,0 | 45,0 |
| Acetat⁻ | [mmol / l] | 4,05 | 26,5 | 40,0 |
| Harnstoff | [mmol / l] | 1,00 | 0,00 | 0,00 |
| NH₄⁺ | [mmol / l] | 0,05 | 0,00 | 0,00 |
| EDTA⁽⁴⁻⁾ | [mmol / l] | 0,50 | 0,50 | 0,50 |
| pH (37°C) | [pH-units] | 7,38 | 7,38 | 7,38 |

**Tabelle 8**

| Wässrige Lösung mit 120 µM Creatinin und 210 µM Creatin | Creatinin-sensor mit Acetat-Zusatz kalibriert | Creatmin-sensor ohne Acetat-Zusatz kalibriert | Differenz | Creatinsensor mit Acetat-Zusatz kalibriert | Creatinsensor ohne Acetat-Zusatz kalibriert | Differenz |
|---|---|---|---|---|---|---|
| Ohne Interferenten | 234 µM Creatinin | 121 µM | 48 % | 363 µM Creatin | 209 µM | 42 % |
| + 40 mmol/l Acetat | 124 µM | 68 µM | 45 % | 211 µM | 133 µM | 37 % |
| + 25 mmol/l Bicarbonat | 152 µM | 80 µM | 47 % | 266 µM | 161 µM | 39 % |
| Durchschnitt | | | 47% | | | 40 % |

**Tabelle 9**

| Wässrige Lösung mit 230 µM Creatinin | Creatininsensor mit Acetat-Zusatz kalibriert | Creatininsensor ohne Acetat-Zusatz kalibriert | Differenz |
|---|---|---|---|
| Ohne Interferenten | 371 µM Creatinin | 225 µM | 39 % |
| + 40 mmol/l Acetat | 229 µM | 146 µM | 36 % |
| + 25 mmol/l Bicarbonat | 226 µM | 137 µM | 39 % |
| Durchschnitt | | | 38 % |

**Tabelle 10**

| Kontrollmittel | Creatiningehalt *des Kontroll-* mittels | Creatingehalt *des Kontroll-* mittels | Creatininsensor *mit Acetat-* Zusatz kalibriert | Creatininsensor *ohne Acetat-* Zusatz kalibriert | Creatinsensor *mit Acetat-* Zusatz kalibriert | Creatinsensor *ohne Acetat-* Zusatz kalibriert |
|---|---|---|---|---|---|---|
| Rinderserum | 97 µM | - | 72 µM | 34 µM | 249 µM | 157 µM |
| Autotrol 1 | 530 µM | 729 µM | 425 µM | 255 µM | 619 µM | 412 µM |
| Autotrol 2 | 176 µM | 242 µM | 138 µM | 82 µM | 220 µM | 137 µM |
| Autotrol 3 | 88 µM | 125 µM | 70 µM | 43 µM | 116 µM | 80 µM |

## Patentansprüche

1. Verfahren zur Kalibration eines Biosensors zur amperometrischen Bestimmung von Creatiain in biologischen Flüssigkeiten, bestehend aus jeweils einem Elektrodensystem zur Messung der Creatinin- und Creatinkonzentration, wobei mindestens zwei Kalibrationslösungen benutzt werden, **dadurch gekennzeichnet, daß** das Elektrodensystem zur Messung der Creatininkonzentration mit einer Creatinin-Lösung kalibriert wird, welche vor der Kalibrationsmessung in Form einer sauren Lösung mit einer alkalischen Pufferlösung gemischt wurde, und das Elektrodensystem zur Messung der Creatinkonzentration mit einer Lösung kalibriert wird, in der sich Creatinin und Creatin im thermodynamischen Gleichgewicht befinden.

2. Verfahren nach Anspruch 1, **dadurch gekennzeichnet, daß** als saure Lösung eine Creatinin-Lösung mit einem pH-Wert von 2-4, vorzugsweise 2,5-3, eingesetzt wird.

3. Verfahren nach Anspruch 1 oder 2, **dadurch gekennzeichnet, daß** die genaue Creatininkonzentration der Kalibrationslösung nach dem Mischen von Creatinin-Lösung und Pufferlösung mit Hilfe eines Leitions, das nur in einer der beiden Mischungskomponenten eingewogen wurde, berechnet wird.

4. Verfahren nach einem der Ansprüche 1 bis 3, **dadurch gekennzeichnet, daß** den Kalibrationslösungen in den zu untersuchenden Flüssigkeiten vorhandene Interferenten, beispielsweise Enzyminhibitoren, und/oder Inhibitoren, welche eine ähnliche Wirkung auf die Enzyme haben wie die in den zu untersuchenden Flüssigkeiten vorhandenen, in deren durchschnittlicher physiologischer Konzentration zugesetzt werden.

5. Verfahren nach Anspruch 4, **dadurch gekennzeichnet, daß** den Kalibrationslösungen Acetat zugesetzt wird.

6. Verfahren nach Anspruch 4, **dadurch gekennzeichnet, daß** den Kalibrationslösungen Bicarbonat zugesetzt wird.

7. Verfahren nach einem der Ansprüche 1 bis 6, **dadurch gekennzeichnet, daß** die Kalibrationslösungen mit Bioziden, wie Isothiazolon-Derivaten, und/oder mit einer Kombination von Bioziden und Antibiotika versetzt werden.

8. Verfahren nach einem der Ansprüche 1 bis 7, **dadurch gekennzeichnet, daß** als Puffer für alkalische Pufferlösungen Amin-Puffer nach Norman Good oder Bicarbonat eingesetzt werden.

9. Verfahren nach einem der Ansprüche 1 bis 7, **dadurch gekennzeichnet, daß** als Puffer für saure Lösungen Amin-Puffer mit Sulfonsäuregruppen nach Norman Good oder Lactat/Milchsäure eingesetzt werden.

## Claims

1. A method for calibrating a biosensor for the amperometric determination of creatinine in biological liquids, consisting of one electrode system each for measuring the concentrations of creatinine and creatine, with at least two calibration solutions being used, **characterized in that** the electrode system for measuring the creatinine concentration is calibrated with a creatinine solution which, prior to the calibration measurement, was mixed in the form of an acidic solution with an alkaline buffer solution, and **in that** the electrode system for measuring the creatine concentration is calibrated with a solution in which creatinine and creatine are at a thermodynamic equilibrium.

2. A method according to claim 1, **characterized in that** a creatinine solution having a pH of from 2 to 4, preferably from 2.5 to 3, is used as the acidic solution.

3. A method according to claim 1 or 2, **characterized in that**, upon mixing the creatinine solution and the buffer solution, the exact creatinine concentration of the calibration solution is calculated by the aid of a ionic tracer only weighed into one of the two mixing components.

4. A method according to any of claims 1 to 3, **characterized in that** interfering substances present in the liquids to be examined, for example, enzyme inhibitors and/or inhibitors having a similar effect on the enzymes such as those present in the liquids to be examined, are added to the calibration solutions at their average physiological concentrations.

5. A method according to claim 4, **characterized in that** acetate is added to the calibration solutions.

6. A method according to claim 4, **characterized in that** bicarbonate is added to the calibration solutions.

7. A method according to any of claims 1 to 6, **characterized in that** the calibration solutions are mixed with biocides such as isothiazolone derivatives, and/or a combination of biocides and antibiotics.

8. A method according to any of claims 1 to 7, **characterized in that** amine buffers according to Norman Good or bicarbonate are used as buffers for alkaline buffer solutions.

9. A method according to any of claims 1 to 7, **characterized in that** amine buffers comprising sulfonic acid groups according to Norman Good or lactate/lactic acid are used as buffers for acidic solutions.

## Revendications

1. Procédé d'étalonnage d'un biocapteur pour la détermination ampérométrique de créatinine dans des liquides biologiques, comprenant respectivement un système d'électrodes pour mesurer la concentration de créatinine et de créatine, au moins deux solutions d'étalonnage étant utilisées, **caractérisé en ce que** le système d'électrodes pour mesurer la concentration de la créatinine est étalonné avec une solution de créatinine qui a été mélangée avant la mesure de l'étalonnage sous la forme d'une solution acide avec une solution tampon alcaline, et le système d'électrodes pour la mesure de la concentration de créatine est étalonné avec une solution dans laquelle la créatinine et la créatine se trouvent en équilibre thermodynamique.

2. Procédé selon la revendication 1, **caractérisé en ce que**, comme solution acide, on utilise une solution de créatinine avec un pH de 2 à 4, de préférence de 2,5 à 3.

3. Procédé selon la revendication 1 ou 2, **caractérisé en ce que** la concentration exacte de créatinine de la solution d'étalonnage est calculée après le mélange de la solution de créatinine et de la solution tampon à l'aide d'un ion conducteur qui a été placé uniquement dans l'un des deux composants de mélange.

4. Procédé selon l'une quelconque des revendications 1 à 3, **caractérisé en ce que** l'on ajoute aux solutions d'étalonnage des interférents présents dans les liquides à analyser, par exemple des inhibiteurs enzymatiques et/ou des inhibiteurs qui exercent un effet comparable sur les enzymes comme celles qui sont présentes dans les liquides à analyser, dans leur concentration physiologique moyenne.

5. Procédé selon la revendication 4, **caractérisée en ce que** l'on ajoute de l'acétate aux solutions d'étalonnage.

6. Procédé selon la revendication 4, **caractérisée en ce que** l'on ajoute du bicarbonate aux solutions d'étalonnage.

7. Procédé selon l'une quelconque des revendications 1 à 6, **caractérisé en ce que** l'on ajoute aux solutions d'étalonnage des biocides, tels que des dérivés d'isothiazolone, et/ou une combinaison de biocides et d'antibiotiques.

8. Procédé selon l'une quelconque des revendications 1 à 7, **caractérisé en ce que** l'on utilise comme tampon pour les solutions tampons alcalines un tampon amine selon Norman Good ou du bicarbonate.

9. Procédé selon l'une quelconque des revendications 1 à 7, **caractérisé en ce que** l'on utilise comme tampon pour les solutions acides un tampon amine avec des groupes d'acide sulfonique selon Norman Good ou du lactate/ acide lactique.
